# EUROPEAN PATENT SPECIFICATION

(11) **EP 2 452 681 B1**
(45) Date of publication and mention of the grant of the patent: **15.03.2017**
(21) Application number: 10796739.0
(22) Date of filing: 09.07.2010
(51) Int. Cl.: A61K 31/437, C07D 471/04, A61K 9/00, A61K 9/08, A61K 9/16, A61K 9/20, A61K 9/48, A61P 35/00, A61P 35/04, A61P 29/00

(54) **SALTS OF 13a-(S)-DEOXYTYLOPHORININE, PREPARATION METHODS AND PHARMACEUTICAL COMPOSITIONS AND USES THEREOF**
SALZE VON 13a-(S)-DEOXYTYLPHORININ, VERFAHREN ZU IHRER HERSTELLUNG SOWIE PHARMAZEUTISCHE ZUSAMMENSETZUNGEN DARAUS UND VERWENDUNGEN DAVON
SELS DE LA 13a-(S)-DÉOXYTYLOPHORININE, LEURS MÉTHODES DE PRÉPARATION, COMPOSITIONS PHARMACEUTIQUES ET UTILISATIONS

(30) Priority: 09.07.2009 CN 200910089238
(43) Date of publication of application: 16.05.2012
(73) Proprietor: Institute of Mataria Medica, Chinese Academy of Medical Sciences, Beijing 100050 (CN)
(72) Inventor: YU, Shishan, Beijing 100050 (CN); CHEN, Xiaoguang, Beijing 100050 (CN); LV, Haining, Beijing 100050 (CN); LI, Yan, Beijing 100050 (CN); XU, Song, Beijing 100050 (CN); MA, Shuanggang, Beijing 100050 (CN); LIU, Zhenjia, Beijing 100050 (CN); ZHANG, Yi, Beijing 100050 (CN); HU, Jinping, Beijing 100050 (CN)
(74) Representative: Nevant, Marc
(86) International application number: PCT/CN2010/075083
(87) International publication number: WO 2011/003362

(56) References cited:
- CN-A- 101 058 578
- CN-A- 101 189 968
- WANG KAILIANG ET AL: "Synthesis and antiviral activities of phenanthroindolizidine alkaloids and their derivatives.", JOURNAL OF AGRICULTURAL AND FOOD CHEMISTRY 10 MAR 2010 LNKD- PUBMED:20000413, vol. 58, no. 5, 10 March 2010 (2010-03-10) , pages 2703-2709, XP002686529, ISSN: 1520-5118
- CHENG-WEI YANG ET AL.: 'Anti-inflammatory Mechanisms of Phenanthroindolizidine alkaloids.' MOL PHARMACOL vol. 69, no. 3, 2006, pages 749 - 758, XP008151153
- BASTIN R J ET AL: "Salt Selection and Optimisation for Pharmaceutical New Chemical Entities", ORGANIC PROCESS RESEARCH AND DEVELOPMENT, CAMBRIDGE, GB, vol. 4, no. 5, 1 January 2000 (2000-01-01) , pages 427-435, XP002228592, DOI: 10.1021/OP000018U

## Description

### TECHNICAL FIELD

The present invention relates to salts of 13a-(S)-deoxytylophorinine, pharmaceutical compositions containing them, and their use as medicaments for the prevention or treatment of cancer or inflammation diseases.

### BACKGROUND ART

Phenanthroindolizidine alkaloids are pentacyclic natural products isolated mainly in plants belonging to *Tylophora* as well as some genera of the *Asclepiadaceae*. These natural products attract widespread attention for their extensively therapeutic activities, especially anti-tumor activity and anti-inflammatory activity. Some of these alkaloids have been screened against 60 tumor cell lines by NCI of USA. It was found that some of these compounds display highly cytotoxic activity with GI₅₀ less than 10⁻⁸M. Furthermore, this activity exhibit highly selectivity to some malignant tumor cells, such as melanoma and lung cancer. These alkaloids are effective to drug-fast cancer cells without cross resistance with other anti-cancer drug.

(+)-13a-(S)-Deoxytylophorinine (Chinese patent application No. 200610076298 X; CN101058578), one of the phenanthroindolizidine alkaloids, was isolated from *Tylophora atrofoculata* and *Tylophora ovata*, and exhibits notable anti-tumor activity with an IC₅₀ to Ketr3, HCT-8, A549, BGC-803, Bel-7402, B16BL6, KB, CaSE-17, HL-60 cells from 0.1 to 0.3µM. (+)-13a-(S)-Deoxytylophorinine displayed antitumor activity in vitro. It exhibited notable anti-tumor proliferative activity of mice liver cancer (H22) and Lewis lung cancer and displayed good antitumor activity *in vivo*. The compound is not toxic in the liver and kidney in preliminary toxic experiments.

Studies of the inhibitory mechanisms illustrated that phenanthroindolizidine alkaloids could inhibit RNA, DNA and protein synthesis (Bioorg Med Chem Lett. 2006, 16: 4300-4304.). But further studies indicated that the mechanism of action of these compounds is completely different from clinically used anti-tumor drugs. These compounds can intensively inhibit NF-κB signal pathway that participate in RNA transcription procedure selectively (Mol Cancer Ther. 2006, 5(10):2484-2493.). But until now the specific target is still unknown.

The anti-inflammation activity of these alkaloids is also related with their inhibition to NF-κB signal pathway, which intensively correlate with the action that blocks MEKK1 action (Mol Pharm. 2006, 69(3):749-758). But the specific target is unknown.

### SUMMARY OF THE INVENTION

The technological problem solved by the present invention is to provide salts of a compound of general formula (I).

Another technological problem solved by the present invention is to provide pharmaceutical compositions comprising at least one salt of a compound of general formula (I), and a pharmaceutical carrier and/or excipient.

Another technological problem solved by the present invention is to provide salts of a compound of general formula (I) for use in the prevention or treatment of cancer or inflammation diseases.

To solve the said above problems, the technological program the present invention applied is:
According to the present invention, salts of (+)-13a-(S)-Deoxytylophorinine are represented by general formula (I): 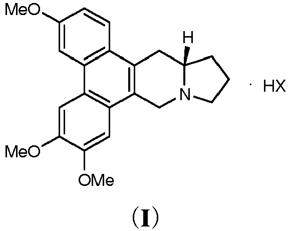 Wherein:
   HX is an organic acid selected from tartaric acid, citric acid and maleic acid.
      (+)-13a-(S)-Deoxytylophorinine salts of the invention are represented by the following formulae: 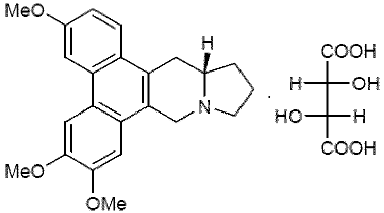 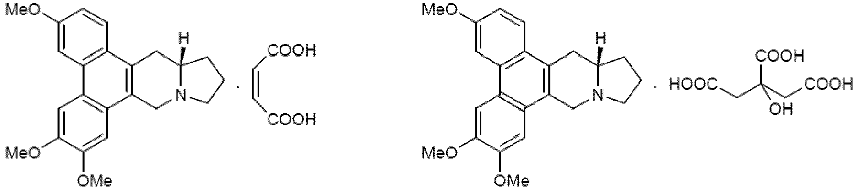
The compounds of the invention can be prepared as follows:

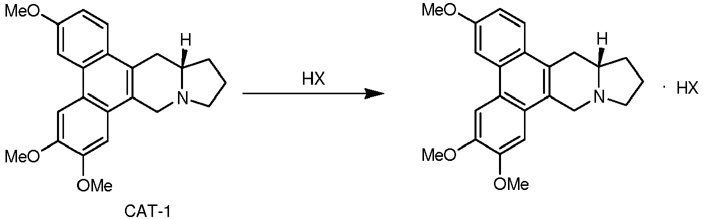

Details of the preparation method include several procedures below:
Equivalent (+)-13a-(S)-Deoxytylophorinine (CAT-1) and corresponding acid were suspended in anhydrous ethanol. The mixture was stirred at 50°C for 30 min until it became clear. The reactant solvent was cooled to room temperature and evaporated to give a solid salt.

The salt prepared can be purified by recrystallization from ethanol.
(+)-13a-(S)-Deoxytylophorinine (CAT-1) can be prepared according to the method of "13a-(S) -deoxytylophorinine, preparation methods, compositions, and uses" (Chinese patent application No. 200610076298.X) and "13a-(S) -deoxytylophorinine analogue, preparation methods, compositions, and uses" (Chinese patent application No. 200910079163.2).

The present invention provides a pharmaceutical composition comprising a therapeutically effective amount of (+)-13a-(S)-Deoxytylophorinine salt of formula (I) and a pharmaceutically acceptable carrier.

The present invention relates to a pharmaceutical composition containing a compound of the invention as active component, and a pharmaceutical excipient or adjuvant. The normal content of the compound of the invention in the pharmaceutical composition of invention is 0.1-95% (g/g). The normal content of the compound of the invention in a unit dosage form is 0.1-100mg, the optimized content is 4-50mg.

The pharmaceutical composition can be prepared according to customary methods. For this reason, if required, the compound of the invention can be prepared as human or veterinary medicine combined with one or more solid or liquid pharmaceutically acceptable excipient and/or adjuvant as administering form or dosage form.

The compound of the invention or its pharmaceutical composition can be administered as a unit dosage form. The administering route can be intestinal tract or others, such as muscle, subcutaneous tissue, nasal cavity, oral mucosa, skin, abdominal membrane, intestinum rectum.

The compound of the invention or its pharmaceutical composition can be administered by injection, including intravenous injection, intramuscular injection, hypodermic injection, endodermic injection, injectio ad acumen.

The administration form can be presented as a liquid or solid form. The liquid form can be a solution, colloid, particulate, emulsion, suspension. Others can be present in the form of tablets, capsules, drop pills, aerosols, pills, powder, solution, suspension, emulsion, granules, suppositories, freeze-dried injectable powder, etc.

The compound of the present invention can be prepared as general preparation, and can also be prepared as sustained release preparation, controlled release preparation, targeting preparation, and all kinds of particulate delivery system.

General customary pharmaceutical carriers can be used in the preparation of tablets from a unit administering dosage form of the compound of invention. Particular examples of the carrier, such as diluent agent and absorption agent are starch, dextrin, calcium sulfate, lactose, mannitol, sucrose, sodium chloride, glycose, urea, calcium carbonate, porcellanite, microcrystalline cellulose, aluminium silicate, etc. Particular examples of wetting agent and adhesive are water, glycerol, polyglycol, ethanol, propanol, starch paste, dextrin, syrup, honey, glycose solution, acacia mucilage, gelatin mucilage, CMC-Na, shellac, methyl cellulose, tribasic potassium phosphate, plasdone, etc. Particular examples of disintegrating agent are dry starch, alginate, powdered agar, laminarin, bicarbonate of soda and citric acid, calcium carbonate, fatty acid ester of polyoxyethylene sorbitan, sodium dodecylsulphate (SDS), methyl cellulose, ethyl cellulose, etc. Particular examples of disintegrating depressor agent are sucrose, tristearin, cacao butter, hydrogenated oil, etc. Particular examples of absorption enhancer are quaternary ammonium salt, sodium dodecylsulfate, etc. Particular examples of lubricant are chalk, silicon dioxide, corn starch, stearate, boracic acid, liquid paraffin, polyglycol, etc. The tablet can be further prepared as coated tablet, such as sugar coated tablet, film coated tablet, enteric-coated tablet, double layer tablet and poly layer tablet.

General customary pharmaceutical carriers can be used in the preparation of pills from a unit administering dosage form of the compound of invention. Particular examples of the carrier, such as diluent agent and absorbent are glycose, lactose, starch, cacao butter, hydrogenated vegetable oil, PVP, glycerolipid ester of monostearate, kaolin, chalk, etc. Particular examples of bonding agent are acacia, gum dragon, gelatin, ethanol, honey, liquid sugar, panada, etc. Particular examples of disintegrating agent are powdered agar, dry starch, alginate, sodium dodecylsulphate (SDS), CMC-Na, methyl cellulose, methyl cellulose, ethyl cellulose, etc.

To prepare capsules as unit dosage form, the mixture of active compound of the present invention and the pharmaceutical carrier mentioned above can be filled in hard gelatin capsule or soft capsule after well-distributed blending. The active compound could also fiat microcapsule and suspended in aqueous medium as suspension, or filled in hard capsule, or as injection.

The compound of the present invention can be prepared as injection, such as solution, suspension, emulsion, freeze-dried injection powder. The preparation can be aqueous or non-aqueous and comprise one or more pharmaceutically acceptable carrier, diluent agent, bonding agent, lubricant, preservative, surfactant or dispersing agent. Particular examples of diluent agent are water, ethanol, polyglycol, trimethylene glycol, ethoxyl-isooctadecanol, polyoxide of isooctadecanol, fatty acid ester of polyoxyethylene sorbitan, etc. Sodium chloride, glucose and glycerol can be added to the injection to make it isotonic. The preparation can also contain one or more agents such as auxiliary solvent, buffer, pH regulator, which are customary used in this field.

Some other agents can be added to the preparation such as colorings, preservatives, flavoring, correctant, sweeteners or others if required.

To achieve and reinforce the therapeutic efficacy, the compound and composition of present invention could be administered by any known route.

The dose of the compound and composition of the present invention depend on many factors, such as the kind and severity of the disease, gender, age, weight, character and individual response of the patients or animals, administering route, administering frequency and goals of treatment. Therefore the administering dose of the compound and composition could be changed in a large scope. Generally say, the dosage of active component of the present invention is generally accepted by the technicians of this field. In order to meet the requirement of efficacy and achieve the prophylactic or therapeutic purpose of the present invention, the administering dose could be adjusted according to the actual content of the compound in the composition of the present invention. The suitable dosage range of the compound of the invention is 0.001-100mg/Kg body weight per day, the optimized dose is 0.1-60mg/Kg body weight, the better choice is 1-30mg/Kg body weight, the best choice is 2-15mg/Kg body weight. The suitable dosage range of the compound of the invention for an adult patient is 10-500mg per day, the optimized dose is 20-100mg, and can be administered in one time or divided into 2-3 times. The suitable dosage range for a child patient is 5-30mg/Kg body weight, the optimized dose is 10-20mg/kg body weight. The compound of the said dose above can be administered by a single dose pattern or divided into two-, three- or four-dose pattern, which is limited by the clinic experiences and administering program of the doctor. The compound of the present invention can be administered alone, or combined with other drugs.

The present invention relates to the application of the compound of the invention in preventing or treating cancer or inflammation diseases. The particular examples of said cancer comprising human colon cancer, human stomach cancer, human ovarian cancer, human uterine cervix cancer, human liver cancer, human lung cancer, human pancreatic cancer.

The technological effect of present invention is:
(+)-13a-(S)-Deoxytylophorinine cannot be dissolved in water due to its high liposolubility. The water-solubility is elevated after salification.

The anti-tumor activity of (+)-13a-(S)-Deoxytylophorinine salts is equivalent to (+)-13a-(S)-Deoxytylophorinine according to the result of *in vitro* activity assay and pharmacodynamics experiment, which means the anti-tumor activity is retained after salification.

As revealed by the results of plasma pharmacokinetics study with the salts of tartaric acid, citric acid and maleic acid, the pharmacokinetics qualities of (+)-13a-(S)-Deoxytylophorinine was optimized with faster absorption, shorter peak time. Moreover, the *in vivo* MRT of the salts was obviously longer than (+)-13a-(S)-Deoxytylophorinine. The AUC of the salts such as maleic acid salt was equivalent to or higher than that of (+)-13a-(S)-Deoxytylophorinine. After oral administration of the tartrate, citrate and maleate, the contents of tartrate and maleate in experimental animal brain tissue were higher than their prototype at three time points.

### DESCRIPTION OF THE FIGURES

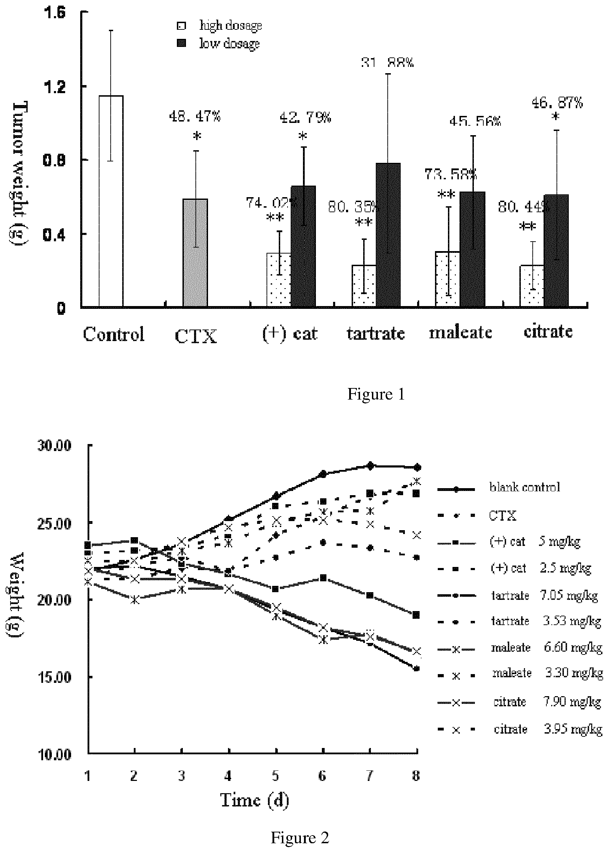

Figure 1: Impact of (+)-13a-(S)-Deoxytylophorinine and its salts on the growth of Cancer Xenografts (H22) in Mice.
Figure 2: Impact of (+)-13a-(S)-Deoxytylophorinine and its salt on the body weight of Kunming (KM) mice.
Figure 3: Impact of (+)-13a-(S)-Deoxytylophorinine and its salt on the growth of Cancer Xenografts (H22) in Mice.
Figure 4: Standard curve of (+)-13a-(S)-Deoxytylophorinine in mice plasma
Figure 5: Concentration-time curve of (+)-13a-(S)-Deoxytylophorinine and its three salts (6 mg/kg) in plasma after oral administration.
Figure 6: Contents of (+)-13a-(S)-Deoxytylophorinine tartrate (6 mg/kg) in brain tissue after oral administration.
Figure 7: Contents of (+)-13a-(S)-Deoxytylophorinine maleate (6 mg/kg) in brain tissue after oral administration.
Figure 8: Contents of (+)-13a-(S)-Deoxytylophorinine citrate (6 mg/kg) in brain tissue after oral administration.

### EXAMPLES

The used starting chemical compounds of the detailed description of the present invention can be prepared by customary methods that are well known for the technicians of this field, and can be prepared by the method of description below.

The purpose of the detailed description below is to illustrate the present invention further, without entailing any limitations to this invention.

### Abbreviations:

CTX : Cytoxan
LC/MS/MS: liquid chromatography-mass spectroscopy
DMSO : Dimethyl Sulfoxide
MRT(0-∞): Mean residence time
AUC(0-∞) : Biological availability
t1/2z : Half-life period
Tmax : Peak Time
Cmax : Peak concentration
+CAT: (+)-13a-(S)-Deoxytylophorinine

### Synthesis example: synthesis of (+)-13a-(S)-deoxytylophorinine.

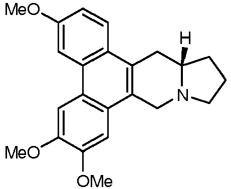

(+)-13a-(S)-deoxytylophorinine was synthesized according to the method of patent « 13a-(S) -deoxytylophorinine, its preparation, pharmaceutical formulations and application » (application number 200610076298.X) and « 13a-(S) -deoxytylophorinine analogue, its preparation, pharmaceutical formulations and application» (application number 200910079163.2). [α]²⁰,_{D} (*c*=0.25,CHCl₃)= +102° , ee = 99.1% [AD-H; isopropanol: hexane (15: 85), 0.1% TEA; λ= 254nm; *t*(major) =18.79min, *t*(minor) = 26.09 min], ESI-MS: 364.2 [M+H]⁺, ¹H-NMR (400 MHz, CDCl₃): 7.92(1H, d, J= 9.2Hz, H-1), 7.21(1H, dd, J= 9.2Hz, 2Hz, H-2), 7.88(1H, d, J= 2Hz, H-4), 7.90(1H, s, H-5), 7.12(1H, s, H-8), 4.00(3H, s, MeO), 4.04(3H, s, MeO), 4.09(3H, s, MeO), 4.62(1H, d, J= 14.8Hz, H-9), 3.71(1H, d, J= 14.8Hz, H-9), 3.45(1H, m, H-10), 3.39(1H, m, H-10), 2.98(1H, m, H-14), 2.57(1H, m, H-13a), 2.53(1H, m, H-10), 2.24(1H, m, H-12), 2.04(1H, m, H-11), 1.93(1H, m, H-11), 1.78(1H, m, H-12). ¹³C-NMR(100 MHz, CDCl₃): 125.11(C-1), 114.83(C-2), 157.61(C-3), 104.55(C-4), 103.92(C-5), 149.42(C-6), 148.30(C-7), 103.02(C-8), 53.46(C-9), 54.91(C-10), 21.53(C-11), 31.03(C-12), 60.15(C13a), 33.03(C-14), 55.48(C3-OMe), 55.96(C6-OMe), 55.90(C7-OMe), 123.34, 125.41, 125.42, 126.78, 130.39. HRMS (ESI) calcd for [M]⁺ C₂₃H₂₆NO₃ 363.1834, found 363.1852.

### Example 1

### Preparation of (+)-13a-(S)-deoxytylophorinine salt (CAT-1 Tartrate)

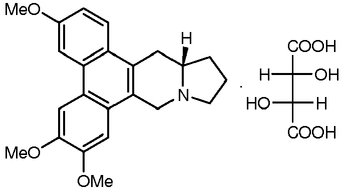

20 mg of (+)-13a-(S)-Deoxytylophorinine and 8.26 mg of tartaric acid were suspended in 5mL of ethanol. The mixture was stirred for 10 min in reflux .The reactant solvent was cooled to room temperature and evaporated to 2mL with a white solid precipitating. After filtration 22 mg of the tartrate salt were obtained. Mp: 216-218°C. The product was soluble in water.

### Example 2

### Preparation of (+)-13a-(S)-deoxytylophorinine salt (CAT-1 Maleate)

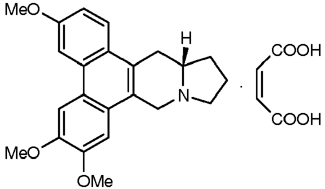

50 mg of (+)-13a-(S)-Deoxytylophorinine and 16 mg of maleic acid were suspended in 10 mL of ethanol. The mixture was stirred for 10 min in reflux until becoming clear. The reactant solvent was cooled to room temperature and evaporated to 5 mL with yellow solid precipitating. After filtration 45 mg of the maleate salt were obtained. Mp: 126-128°C. The product was soluble in water.

### Example 3

### Preparation of (+)-13a-(S)-deoxytylophorinine salt (CAT-1 Citrate)

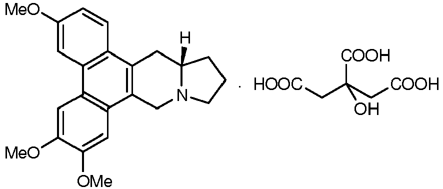

50 mg of (+)-13a-(S)-Deoxytylophorinine and 29 mg of citric acid were suspended in 10 mL of ethanol. The mixture was stirred for 10 min in reflux until becoming clear. The reactant solvent was cooled to room temperature and evaporated to 5 mL with a yellow solid precipitating After filtration 60 mg of the citrate salt were obtained. Mp: 142-144°C. The product was soluble in water.

### Example 4 (not according to the invention)

### Preparation of (+)-13a-(S)-deoxytylophorinine salt (CAT-1 Lactate)

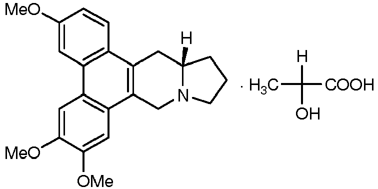

50 mg of (+)-13a-(S)-Deoxytylophorinine and 14 mg of lactic acid were suspended in 10 mL of ethanol. The mixture was stirred for 10 min in reflux until becoming clear. The reactant solvent was cooled to room temperature and evaporated to 5 mL with a yellow solid precipitating. After filtration 45 mg of the lactate salt were obtained. Mp: 115-117°C. The product was soluble in water.

### Example 5 (not according to the invention)

### Preparation of (+)-13a-(S)-deoxytylophorinine salt (CAT-1 hydrochloride)

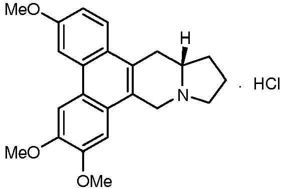

Dry HCl gas was bubbled in a solution of 20mg of (+)-13a-(S)-Deoxytylophorinine in 5mL of dichloromethane for 30 min. The reaction solvent was evaporated to give 20 mg of white solid. Mp: 225-227°C.

### Example 6 (not according to the invention)

### Preparation of (+)-13a-(S)-deoxytylophorinine salt (CAT-1 sulfate)

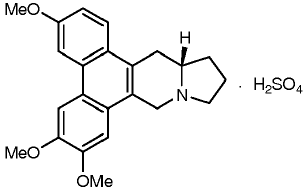

3 mL of 20% (V/V) sulfuric acid in ethanol solution was added dropwise to a mixture of 1 g of (+)-13a-(S)-Deoxytylophorinine in 5 mL of ethanol under stirring. The mixture was heated to reflux until becoming clear and then cooled to room temperature with a white solid precipitating. After filtration 1.1 g of the sulfate salt were obtained. Mp: 175-177°C.

### Solubility in water:

| Sample | +CAT | +CAT Citrate | +CAT Tartrate | +CAT Maleate |
|---|---|---|---|---|
| Solubility | Insoluble | 33 mg / mL | 39 mg / mL | 44 mg / mL |
| Sample | +CAT Lactate* | +CAT Sulfate* | | |
| Solubility | 120 mg / mL | 200 mg / mL | | |

| | | | | |
|---|---|---|---|---|
| * not according to the invention | | | | |

### Pharmacologic Experiments:

### Example 1: Cytotoxic activity assay (MTT)

To evaluate the *in vitro* antitumor activity of (+)-13a-(S)-Deoxytylophorinine salts in this invention, a cytotoxic activity assay (MTT) experiment was carried out.
1. Tumor cells at log phase were incubated on 96-well plates at a density of 1-1.0×10⁴ cells/ml, 100µl/well. Cells were cultured for 24h at 37°C in a cell incubator with 5% CO₂.
2. The treatment cells were exposed to the tested agents at different concentrations and cultured for 5 days in a cell incubator with full humidity incubator.
3. The incubation medium was removed, then 100µl 0.04% MTT was added to each well and the cells were cultured for 4 hours under the same conditions.
4. The incubation medium was removed, then 150µl DMSO was added to each well, the absorbance at a reference wavelength of 450nm and a test wavelength of 570nm was read. The drug inhibitory rate was calculated.

**Table 1: MTT result of 13a-(S)-deoxytylophorinine salts:**

| Samples | IC₅₀ (M) | | | | | | |
|---|---|---|---|---|---|---|---|
| | A549 | Be17402 | U251 | BT325 | A2780 | Bgc823 | Hct8 |
| Tartrate | 6.30× 10⁻⁸ | 6.00× 10⁻⁸ | 1.30× 10⁻⁷ | 5.0× 10⁻⁷ | 8.1× 10⁻⁸ | 6.3× 10⁻⁷ | 7.9× 10⁻⁸ |
| Maleate | 1.40× 10⁻⁷ | 3.20× 10⁻⁸ | 6.30x 10⁻⁸ | 5.9× 10⁻⁷ | 5.0× 10⁻⁸ | 8.9× 10⁻⁷ | 5.8× 10⁻⁸ |
| Lactate* | 1.40× 10⁻⁷ | 4.00× 10⁻⁸ | 9.50× 10⁻⁸ | 7.6× 10⁻⁷ | 1.8× 10⁻⁷ | 1.3× 10⁻⁶ | 7.9× 10⁻⁸ |
| Citrate | 1.30× 10⁻⁷ | 3.70× 10⁻⁸ | 6.90× 10⁻⁸ | 4.1× 10⁻⁷ | 4.9× 10⁻⁸ | 4.0× 10⁻⁷ | 4.1× 10⁻⁸ |
| (+)-13a-(S)-deoxytylophorinine | 4.00× 10⁻⁸ | 3.80× 10⁻⁸ | 7.10× 10⁻⁸ | 5.4× 10⁻⁷ | 5.4× 10⁻⁸ | 5.1× 10⁻⁷ | 5.5× 10⁻⁸ |

| | | | | | | | |
|---|---|---|---|---|---|---|---|
| * not according to the invention | | | | | | | |

A549: human lung cancer cell; Bel-7402: human liver cancer cell; U251: human glioma cell; BT325: human glioma cell, A2780: human ovarian cancer cell, BGC-823: human stomach cancer cell; HCT-8: human colon cancer cell.

The MTT result revealed that the salts of 13a-(S)-deoxytylophorinine displayed notable anti-tumor activity similar to that of the free base.

### Example 2.1

*In vivo* pharmacodynamics experiment of 13a-(S)-deoxytylophorinine and its salts:
To determine the pharmacodynamics characters of 13a-(S)-Deoxytylophorinine and its salts, the impact of these compounds to the tumor growth in H22 tumor-bearing mice was surveyed.

### 1. Experimental animal

Male Kunming (KM) mice were obtained from the Laboratory Animal Center, Academy of Military Medical Science. Body weight 18-22g. 6 animals per group, totally 10 groups, 60 animals.

### 2. Experimental samples and dosage

### (1). Experimental samples

(+)-13a-(S)-Deoxytylophorinine (+CAT), (+)-13a-(S)-Deoxytylophorinine Tartrate,
(+)-13a-(S)-Deoxytylophorinine Maleate, (+)-13a-(S)-Deoxytylophorinine Citrate, Cytoxane (CTX) as positive control.
(+)-13a-(S)-Deoxytylophorinine, MW: 363.45
(+)-13a-(S)-Deoxytylophorinine Tartrate, MW: 513.54
(+)-13a-(S)-Deoxytylophorinine Maleate, MW: 479.52
(+)-13a-(S)-Deoxytylophorinine Citrate, MW: 573.59

### (2) Dosage

(+)-13a-(S)-Deoxytylophorinine was administered at high dose (5mg/kg) and low dose (2.5mg/kg). Three (+)-13a-(S)-Deoxytylophorinine salts were administered at high dose and low dose as equal moles with the free base (Tartrate: 7.05mg/kg, 3.53mg/kg; Malate: 6.60mg/kg, 3.30mg/kg; Citrate: 7.90mg/kg, 3.95mg/kg).

### 3. Method

Animals were bred in SPF grade circumstance to be observed for 24 h. The tumor cells liquid which resuscitated in abdominal cavity of KM mice was diluted with aseptic normal saline at 1:3 ratio. The resulting liquid was inoculated subcutaneously at forelimb (0.2mL to each animal). After the injection, the animals were divided to 12 groups with 6 animals per group.

Four experimental samples were administered intragastrically once daily 24h after inoculation. The positive control CTX was injected intraperitoneally once daily. The animal body weight was recorded every day.

### 4. Result

**Table 2: impact of (+)-13a-(S)-deoxytylophorinine and its salts to the tumor growth of H22 tumor-bearing mice.**

| **Group** | **Dose (mg/kg ×d)** | **Animal No. (Initial/F inal)** | **Body Weight(g) (Initial/Final)** | **Tumor Weight (g, mean±SD)** | **Inhibition (%)** |
|---|---|---|---|---|---|
| Control | | 6/6 | 21.83±1.47 /25.35±2.76 | 1.15±0.35 | |
| CTX (i.p.) | 100×1 | 6/6 | 21.33±1.63 /23.77±1.38 | 0.59±0.26 | 48.47* |
| (+)-13a-(S)-deoxytylophorinine -1 | 5.0×7 | 6/4 | 23.50±2.51 /15.63±2.16 | 0.30±0.12 | 74.02** |
| (+)-13a-(S)-deoxytylophorinine -2 | 2.5×7 | 6/6 | 23.00±1.55 /23.31±2.14 | 0.66±0.21 | 42.79* |
| Tartrate-1 | 7.05x7 | 6/6 | 22.00±1.55 /13.55±1.87 | 0.23±0.15 | 80.35** |
| Tartrate-2 | 3.53x7 | 6/6 | 21.50±0.84 /19.76±2.34 | 0.78±0.48 | 31.88 |
| Maleate-1 | 6.60x7 | 6/4 | 21.17±0.98 /13.62±2.08 | 0.30±0.24 | 73.58** |
| Maleate-2 | 3.30x7 | 6/6 | 22.50±1.05 /23.79±0.52 | 0.62±0.31 | 45.56 |
| Citrate-1 | 7.90x7 | 6/5 | 22.00±1.55 /14.27±2.61 | 0.22±0.13 | 80.44** |
| Citrate-2 | 3.95x7 | 6/6 | 21.83±0.75 /21.01±2.04 | 0.61±0.35 | 46.87* |

| | | | | | |
|---|---|---|---|---|---|
| **p*<0.05, ***p*<0.01 compared with control group. | | | | | |

### 5. Conclusion

The inhibition of tumor growth of animals with the organic acid salts of (+)-13a-(S)-deoxytylophorinine was equal to or higher than their free base at high dose, equal or lower at low dose, but all were higher than the positive control (CTX), which means the anti-tumor activity was retained after salification.

### Example 2.2: In vivo pharmacodynamics experiment (not according to the invention)

1. The impact of 13a-(S)-deoxytylophorinine Sulfate on the tumor growth in H22 tumor-bearing mice was surveyed.
2. Experimental animal
Male Kunming (KM) mice were obtained from the Laboratory Animal Center, Academy of Military Medical Science. Body weight 18~22g.
3. Experimental samples and dosage.
(1). Experimental samples
   (+)-13a-(S)-deoxytylophorinine Sulfate, Cytoxane (CTX) as positive control.
(2) Dosage and administration way
   All the samples were administered orally. o.d. 0.2ml/10g.
   (+)-13a-(S)-deoxytylophorinine Sulfate was administered at five dose groups: 3mg/kg, 4mg/kg, 5mg/kg, 6mg/kg and 8mg/kg.

4. Method
Animals were bred in SPF grade circumstance to be observed for 24 h. The tumor cells liquid which resuscitated in abdominal cavity of KM species mice was diluted with aseptic normal saline at 1:3 ratio. The resulting liquid was inoculated subcutaneously at forelimb (0.2mL to each animal). After the injection, the animals were divided to 13 groups. Experimental samples were administered 24h after inoculation.
5. Result

**Table 1: impact of (+)-13a-(S)-deoxytylophorinine sulfate on the tumor growth of H22 tumor-bearing mice.**

| **Group** | **Dose (mg/kg ×d)** | **Animal No. (Initial/Final)** | **Body Weight(g) (Initial/Final)** | **Tumor Weight (g, mean±SD)** | **Inhibition (%)** |
|---|---|---|---|---|---|
| Control | | 6/6 | 19.67±1.21 /26.17±5.38 | 1.26±0.22 | |
| CTX (i.p.) | 100mg/kgx 1 | 6/6 | 20.67±2.07 /25.00±2.37 | 0.07±0.04 | 94.76** |
| Sulfate (o.p.) | 3mg/kg ×7 | 6/6 | 20.67±0.52 /26.83±2.56 | 0.98±0.21 | 22.49* |
| Sulfate (o.p.) | 4mg/kg ×7 | 6/6 | 20.00±1.26 /22.83±2.40 | 0.60±0.32 | 52.51* |
| Sulfate (o.p.) | 5mg/kg ×7 | 6/6 | 19.83±1.72 /22.17±5.31 | 0.73±0.39 | 41.75* |
| Sulfate (o.p.) | 6mg/kg ×6 | 6/3 | 19.83±1.72 /15.33±3.21 | 0.40±0.24 | 68.52** |
| Sulfate (o.p.) | 8mg/kg ×6 | 6/0 | 20.67±1.03 /--- | 0.15±0.04 | 88.49** |

| | | | | | |
|---|---|---|---|---|---|
| **p*<0.05, ** *p*<0.01 compared with control group. Note: After (+)-13a-(S)-deoxytylophorinine Sulfate (6 mg/kg) was administered orally, one of the mice died on the sixth day, two mice died on the seventh day. The administration was stopped. | | | | | |

After (+)-13a-(S)-deoxytylophorinine Sulfate (8 mg/kg) was administered orally, three mice died on the sixth day, three mice died on the seventh day. The administration was stopped.

The tumor weight data and the inhibition rate of these groups are obtained from dissected mice after (+)-13a-(S)-deoxytylophorinine Sulfate was administered orally for 7 days.

### Example 3: The study about plasma pharmacokinetics and distribution in brain tissue of 13a-(S)-deoxytylophorinine and its salts.

To determine the pharmacokinetic characters of 13a-(S)-deoxytylophorinine and its salts, the studies about plasma pharmacokinetics and the character of distribution in brain were pursued.

### 1. Experimental animal

Male ICR mice. Body weight 18-22g.

### 2. Samples

(+)-13a-(S)-deoxytylophorinine Tartrate, (+)-13a-(S)-deoxytylophorinine Maleate, (+)-13a-(S)-deoxytylophorinine Citrate. All were dissolved in distilled water as 0.6 mg/ml solution before the experiment progressed.

### 3. Method

72 mice were divided into 3 groups with 24 animals per group, fasted for 16h with free access to water. After the salts were administered orally, the blood of mice were collected at 5 min, 15 min, 30 min, 1h, 2h, 3h, 4h, 6h time points. Plasma was isolated by centrifugation. After the salts were administered orally, the brain tissues of mice were collected at 5 min, 15 min, 2h time points. 25% tissue homogenate was prepared with normal saline addition. 200 µL of homogenate and plasma were added to the same volume of MeCN to precipitate the protein. 5 µL of clear supernatant was analyzed by LC/MS/MS.

### 4. Result

### (1). Standard curve

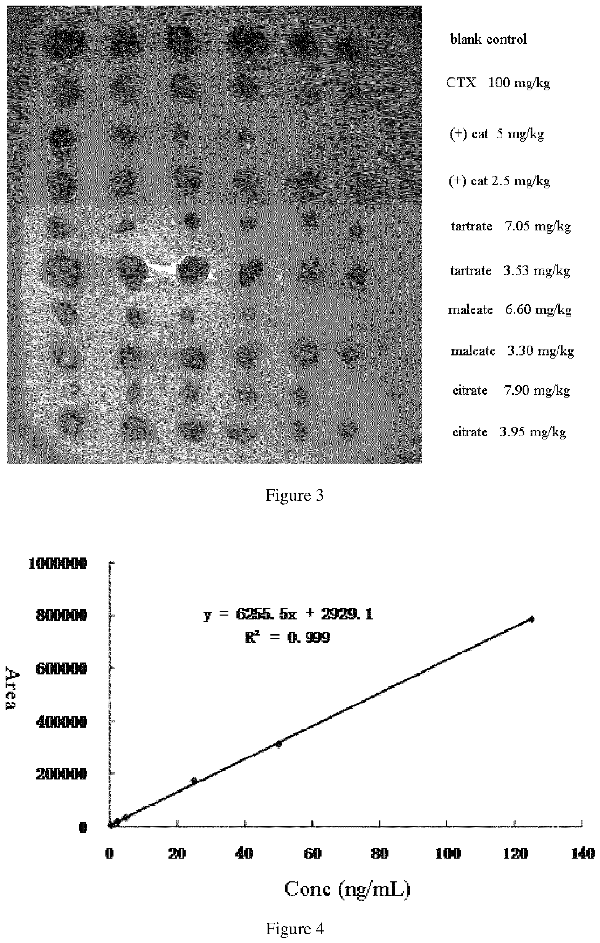

(+)-13a-(S)-deoxytylophorinine was dissolved in DMSO and then diluted with MeOH to concentrations 10, 50, 100, 500, 1000 and 2500 ng/ml respectively. 10 µl of standard solutions of (+)-13a-(S)-deoxytylophorinine with different concentrations above were added to 200 µl of blank plasma in sequence to adjust the concentration to 0.5, 2.5, 5, 25, 50, 125 ng/ml respectively. MeCN was added to the solution to precipitate protein, and 5 µl of result clear supernatant was analyzed with LC/MS/MS. Linear regression was run with the peak area of (+)-13a-(S)-deoxytylophorinine as ordinate and the concentrations as abscissa. The result is represented in Table 3 and Figure 4. In the range of concentration with 0.5-125 ng/ml, the linear relation of the concentrations of (+)-13a-(S)-deoxytylophorinine in plasma and peak area was fine with correlation coefficient 0.999.

**Table 3: standard curve of (+)-13a-(S)-deoxytylophorinine in mice plasma**

| Samples in plasma | |
|---|---|
| Concentration (ng/ml) | Peak area |
| 0.5 | 2707 |
| 2.5 | 16732 |
| 5 | 34240 |
| 25 | 169854 |
| 50 | 310165 |
| 125 | 785030 |

### (2). Plasma pharmacokinetics study of three (+)-13a-(S)-deoxytylophorinine salts (6 mg/kg) after administration orally to mice.

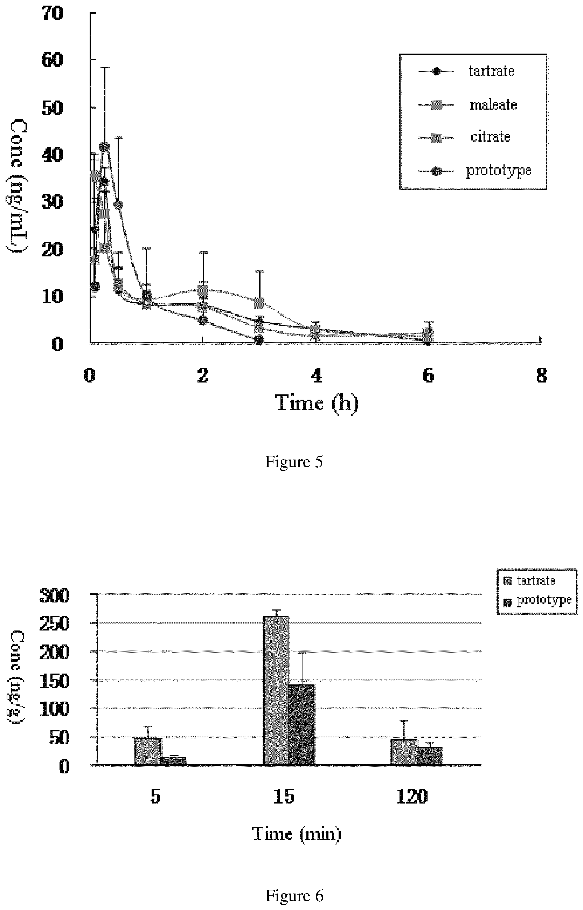

The concentration-time data of (+)-13a-(S)-deoxytylophorinine tartrate, maleate and citrate after administration orally are represented in Tables 4-6 and Figure 5. These salts were absorbed quickly, the free base compound was detected in blood 5 min after oral administration. The peak concentration of tartrate was 34.2±3.1 ng/ml after 15 min. The peak concentration of maleate was 35.4±3.5 ng/ml after 5 min. The peak concentration of citrate was 20.1±12.2 ng/ml after 15 min. The elimination of all the salts were quick with blood drug levels approaching lowest detectable limit after 6 h. The pharmacokinetic parameters are represented in Table 7 which fitted with non-compartment model by using DAS program according to the concentration-time curve of (+)-13a-(S)-deoxytylophorinine salts. MRT (0-∞) of these salts is 1.63-2.29h, which is longer than their free base (0.8h). The AUC(0-∞) of tartrate, maleate and citrate is 37.85, 49.91and 33.08 µg/L*h respectively, compared with their prototype 33.67µg/L*h. The AUC(0-∞) of maleate is higher than other salts and free base compound notably.

**Table 4: drug concentration of (+)-13a-(S)-deoxytylophorinine tartrate administered orally (6 mg/kg, 11.7µM)**

| Time (min) | Blood concentration of (+)-13a-(S)-deoxytylophorinine (ng/ml) | | | |
|---|---|---|---|---|
| | 1 | 2 | 3 | Mean±SD |
| 0.08 | 18.4 | 11.7 | 42.0 | 24.0±15.9 |
| 0.25 | 30.6 | 36.1 | 35.7 | 34.2±3.1 |
| 0.5 | 16.5 | 9.0 | 7.8 | 11.1±4.7 |
| 1 | 6.2 | 10.0 | - | 8.1±2.7 |
| 2 | 2.5 | 12.4 | 8.7 | 7.9±5.0 |
| 3 | 3.0 | 5.0 | 5.3 | 4.5±1.2 |
| 4 | 3.3 | 4.2 | 1.1 | 2.8±1.6 |
| 6 | 0.0 | 0.2 | 0.8 | 0.4±0.4 |

**Table 5: drug concentration of (+)-13a-(S)-deoxytylophorinine maleate administered orally (6 mg/kg, 12.5µM)**

| Time (min) | Blood concentration of (+)-13a-(S)-deoxytylophorinine (ng/ml) | | | |
|---|---|---|---|---|
| | 1 | 2 | 3 | Mean±SD |
| 0.08 | - | 33.0 | 37.9 | 35.4±3.5 |
| 0.25 | 24.2 | 23.5 | 34.6 | 27.4±6.2 |
| 0.5 | 15.7 | 13.0 | 7.9 | 12.2±4.0 |
| 1 | 12.5 | 6.2 | 8.7 | 9.2±3.2 |
| 2 | 2.0 | 15.9 | 15.7 | 11.2±8.0 |
| 3 | 3.6 | 16.1 | 6.3 | 8.6±6.6 |
| 4 | 2.6 | 2.0 | 3.8 | 2.8±0.9 |
| 6 | 2.3 | 1.0 | 0.4 | 1.2±1.0 |

**Table 6: drug concentration of (+)-13a-(S)-deoxytylophorinine citrate administered orally (6 mg/kg, 10.5µM)**

| Time (min) | Blood concentration of (+)-13a-(S)-deoxytylophorinine (ng/ml) | | | |
|---|---|---|---|---|
| | 1 | 2 | 3 | Mean±SD |
| 0.08 | 30.5 | 18.0 | 4.6 | 17.7±13.0 |
| 0.25 | 25.5 | 28.7 | 6.1 | 20.1±12.2 |
| 0.5 | 20.1 | 8.4 | 9.3 | 12.6±6.5 |
| 1 | 11.2 | 5.4 | 8.5 | 8.4±2.9 |
| 2 | 5.6 | 7.6 | 9.6 | 7.6±2.0 |
| 3 | 2.8 | 2.3 | 4.7 | 3.3±1.3 |
| 4 | 1.1 | 0.9 | 2.6 | 1.5±0.9 |
| 6 | 1.4 | 4.8 | 0.1 | 2.1±2.4 |

**Table 7: pharmacokinetic parameters of (+)-13a-(S)-deoxytylophorinine salts administered orally (6 mg/kg)**

| Parameter | Unit | Parameter value | | | |
|---|---|---|---|---|---|
| | | Tartrate | Maleate | Citrate | Prototype |
| AUC(0-t) | µg/L*h | 37.39 | 46.87 | 32.70 | 33.34 |
| AUC(0-∞) | µg/L*h | 37.85 | 49.91 | 33.08 | 33.67 |
| MRT(0-t) | h | 1.57 | 1.78 | 1.73 | 0.77 |
| MRT(0-∞) | h | 1.63 | 2.18 | 2.29 | 0.80 |
| t1/2z | h | 0.88 | 1.72 | 0.86 | 0.44 |
| Tmax | h | 0.25 | 0.08 | 0.25 | 0.25 |
| Cmax | µg/L | 34.17 | 35.45 | 20.10 | 41.53 |

### (3) Drug distribution study of three (+)-13a-(S)-deoxytylophorinine salts in brain tissue after administration orally to mice.

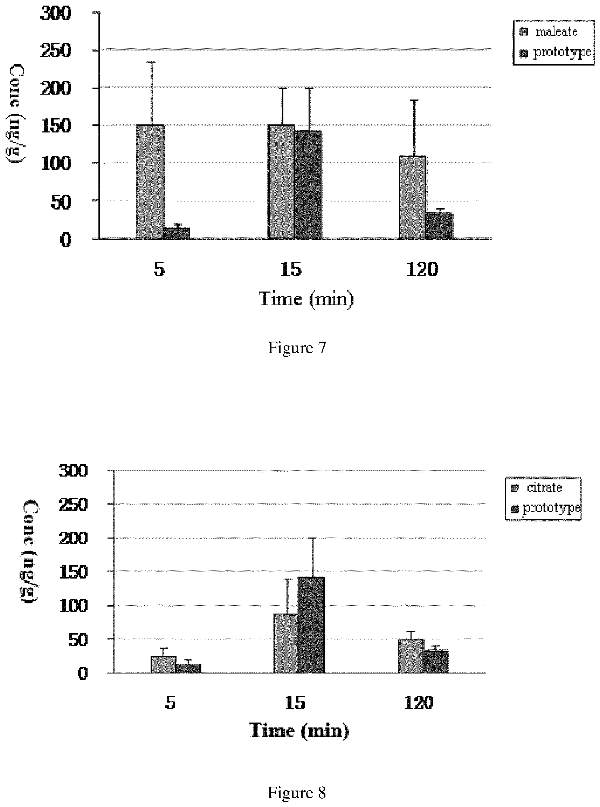

The drug concentration-time data in brain tissue after administered orally of (+)-13a-(S)-deoxytylophorinine tartrate, maleate and citrate are represented in Tables 8-10 and Figures 6-8. As the results revealed, the drug was distributed into brain tissue easily after the salts were taken orally. (+)-13a-(S)-deoxytylophorinine was detected in 5 min. The peak concentration was reached after 15 min, and the compound still could be detected after 2 h after administration. The drug contents of tartrate and maleate in brain at 3 time points were all higher than their free base, and the content of citrate was lower than its free base.

**Table 8: drug content in brain tissue of (+)-13a-(S)-deoxytylophorinine tartrate administered orally (6 mg/kg)**

| Time (min) | Brain contents of (+)-13a-(S)-deoxytylophorinine (ng/ml) | | | |
|---|---|---|---|---|
| | 1 | 2 | 3 | Mean±SD |
| 5 | 32.4 | 38.8 | 72.7 | 48.0±21.6 |
| 15 | 249.3 | 271.0 | 268.5 | 262.9±11.9 |
| 120 | 24.7 | 28.7 | 83.7 | 45.7±33.0 |

**Table 9: drug content in brain tissue of (+)-13a-(S)-deoxytylophorinine maleate administered orally (6 mg/kg)**

| Time (min) | Brain contents of (+)-13a-(S)-deoxytylophorinine (ng/ml) | | | |
|---|---|---|---|---|
| | 1 | 2 | 3 | Mean±SD |
| 5 | 245.2 | 82.5 | 122.7 | 150.2±84.8 |
| 15 | 159.3 | 96.6 | 194.9 | 150.3±49.8 |
| 120 | 27.2 | 173.7 | 125.4 | 108.8±74.7 |

**Table 10: drug content in brain tissue of (+)-13a-(S)-deoxytylophorinine citrate administered orally (6 mg/kg)**

| Time (min) | Brain contents of (+)-13a-(S)-deoxytylophorinine (ng/ml) | | | |
|---|---|---|---|---|
| | 1 | 2 | 3 | Mean±SD |
| 5 | 22.0 | 37.1 | 11.8 | 23.6±12.7 |
| 15 | 84.8 | 138.7 | 38.5 | 87.3±50.1 |
| 120 | 37.0 | 44.8 | 64.4 | 48.7±14.1 |

### 5. Conclusion

The absorption was quick after oral administration of the (+)-13a-(S)-deoxytylophorinine tartrate, maleate and citrate. The peak concentration was 18.0-35.4 ng/ml in 5-30 min. MRT (0-∞) of these salts was 1.63-2.29h, which is longer than their free base (0.8h). The AUC(0-∞) of tartrate, maleate and citrate was 37.85, 49.91and 33.08 µg/L*h respectively, compared with their free base 33.67µg/L*h. The AUC(0-∞) of maleate was higher than other salts and free base compound notably.

The drug was distributed into brain tissue easily after oral administration of the salts. The peak concentration was detected after 15 min, and the compound still could be detected after 2 h after administration. The drug content of both the tartrate and maleate in brain at 3 time points was higher than their free base, and the content of citrate was lower than its free base.

## Claims

1. A salt of (+)-13a-(S)-deoxytylophorinine represented by the general formula (I): 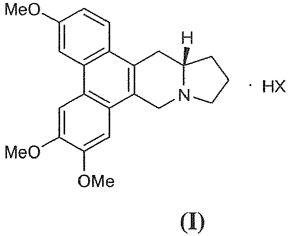 wherein HX is selected from tartaric acid, citric acid and maleic acid.

2. A pharmaceutical composition comprising a therapeutically effective amount of a salt according to claim 1, and a pharmaceutically acceptable carrier.

3. A pharmaceutical composition according to claim 2, **characterized in that** said pharmaceutical composition is selected from tablets, capsules, pills, injections, sustained-release preparations, controlled-release or targeted preparations and various fine particle delivery system.

4. The salt of (+)-13a-(S)-deoxytylophorinine according to claim 1 for use in the prevention or treatment of a cancer.

5. The salt for the use of claim 4, wherein said cancer is selected from human colon cancer, human stomach cancer, human ovarian cancer, uterine cervix cancer, liver cancer, lung cancer, pancreatic cancer, lymphadenoma and glioma.

6. The salt of (+)-13a-(S)-deoxytylophorinine according to claim 1 for use in the prevention or treatment of an inflammation disease.

## Patentansprüche

1. Salz des (+)-13a-(S)-Desoxytylophorinins, das durch die allgemeine Formel (I) dargestellt wird: 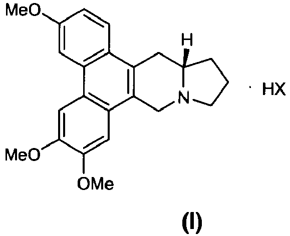 wobei HX aus Weinsäure, Zitronensäure und Maleinsäure ausgewählt ist.

2. Pharmazeutische Zusammensetzung, umfassend eine therapeutisch wirksame Menge eines Salzes nach Anspruch 1 und einen pharmazeutisch akzeptablen Träger.

3. Pharmazeutische Zusammensetzung nach Anspruch 2, **dadurch gekennzeichnet, dass** die pharmazeutische Zusammensetzung aus Tabletten, Kapseln, Pillen, Injektionen, Zubereitungen zur nachhaltigen Freigabe, Zubereitungen zur kontrollierten Freigabe, zielgerichteten Zubereitungen und verschiedenen Feinpartikelabgabesystemen ausgewählt ist.

4. Salz des (+)-13a-(S)-Desoxytylophorinins nach Anspruch 1 zur Verwendung in der Vorbeugung oder Behandlung von Krebs.

5. Salz für die Verwendung nach Anspruch 4, wobei der Krebs aus humanem Darmkrebs, humanem Magenkrebs, humanem Eierstockkrebs, Gebärmutterhalskrebs, Leberkrebs, Lungenkrebs, Bauchspeicheldrüsenkrebs, Lymphadenom und Gliom ausgewählt ist.

6. Salz des (+)-13a-(S)-Desoxytylophorinins nach Anspruch 1 zur Verwendung in der Vorbeugung oder Behandlung einer entzündlichen Erkrankung.

## Revendications

1. Sel de (+)-13a-(S)-désoxytylophorinine représenté par la formule générale (I) : 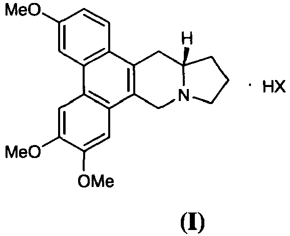 dans laquelle HX est l'acide tartrique, l'acide citrique ou l'acide maléique.

2. Composition pharmaceutique comprenant une quantité thérapeutiquement efficace d'un sel selon la revendication 1, et un véhicule pharmaceutiquement acceptable.

3. Composition pharmaceutique selon la revendication 2, **caractérisée en ce que** ladite composition pharmaceutique est choisie parmi les comprimés, les gélules, les pilules, les injections, les préparations à libération prolongée, les préparations à libération contrôlée, les préparations ciblées et divers systèmes pour délivrer des particules fines.

4. Sel de (+)-13a-(S)-désoxytylophorinine selon la revendication 1 pour son utilisation dans la prévention ou le traitement du cancer.

5. Sel pour l'utilisation selon la revendication 4, où le cancer est choisi parmi le cancer humain du côlon, le cancer humain de l'estomac, le cancer humain des ovaires, le cancer du col de l'utérus, le cancer du foie, le cancer du poumon, le cancer du pancréas, le, lymphadénome et le gliome.

6. Sel de (+)-13a-(S)-désoxytylophorinine selon la revendication 1 pour son utilisation dans la prévention ou le traitement d'une maladie inflammatoire.
